# EUROPEAN PATENT SPECIFICATION

(11) **EP 2 092 901 B1**
(45) Date of publication and mention of the grant of the patent: **16.11.2016**
(21) Application number: 09250461.2
(22) Date of filing: 20.02.2009
(51) Int. Cl.: A61B 17/17

(54) **Guide for creating femoral tunnel during ACL reconstruction**
Führungselement zur Schaffung eines femoralen Tunnels während der ACL-Rekonstruktion
Guide pour la création d'un tunnel fémoral pendant la reconstruction ACL

(30) Priority: 21.02.2008 US 66575 P; 06.02.2009 US 366967
(43) Date of publication of application: 26.08.2009
(73) Proprietor: Covidien LP, Mansfield, MA 02048 (US)
(72) Inventor: Re, Paul, Boston, MA 02116 (US)
(74) Representative: Soames, Candida Jane

(56) References cited:
- EP-A- 1 570 793
- EP-A- 1 714 619
- US-A1- 2002 151 903
- US-A1- 2003 009 173
- US-A1- 2007 191 853

## Description

### BACKGROUND

### Technical Field

This invention relates to surgical apparatus and procedures in general, and more particularly to surgical apparatus and procedures for reconstructing a ligament.

### Background of Related Art

A ligament is a piece of fibrous tissue which connects one bone to another. Ligaments are frequently damaged (e.g., detached or torn or ruptured, etc.) as the result of injury and/or accident. A damaged ligament can cause instability, impede proper motion of a joint and cause pain. Various procedures have been developed to repair or replace a damaged ligament. The specific procedure used depends on the particular ligament which is to be restored and on the extent of the damage.

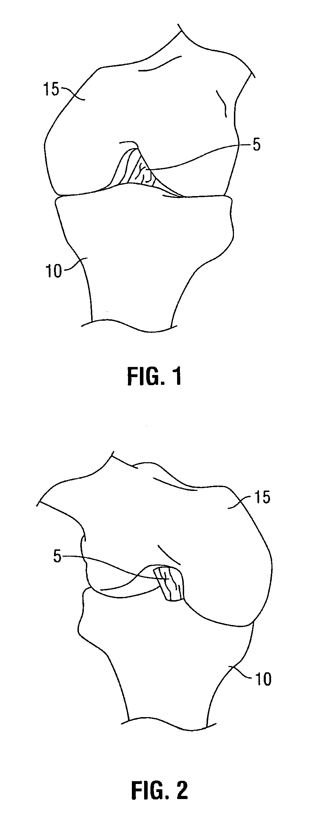

One ligament which is frequently damaged as the result of injury and/or accident is the anterior cruciate ligament (i.e., the ACL). Looking first at Figs. 1 and 2, it will be seen that the ACL 5 extends between the top of the tibia 10 and the bottom of the femur 15. A damaged ACL can cause instability of the knee joint and cause substantial pain and arthritis. For this reason, ACL reconstruction is a common procedure with more than 100,000 cases being performed in the United States annually.

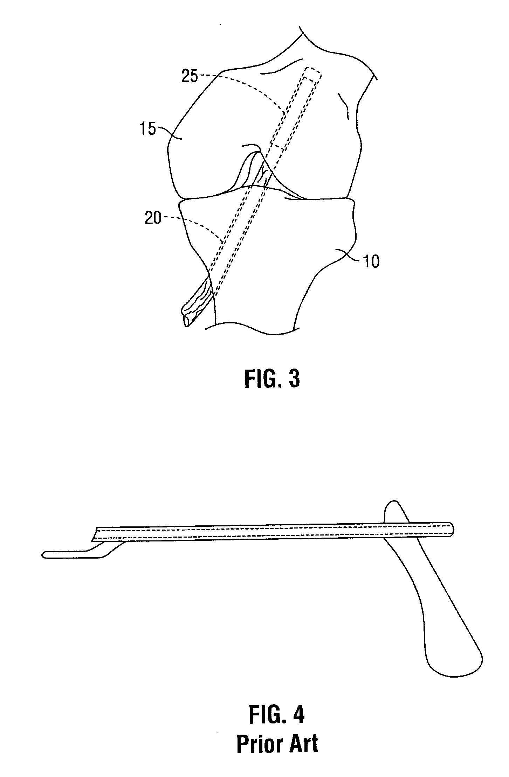

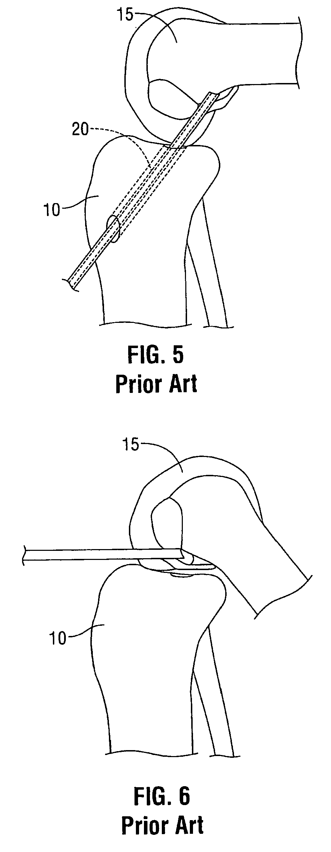

Various procedures have been developed to restore and/or reconstruct a damaged ACL through a graft ligament replacement. Traditionally, this procedure is performed utilizing a trans-tibial approach. In this approach, a tibial tunnel or bone tunnel 20 is created in tibia 20 by drilling up through tibia 10. Bone tunnel 20 is then used to access an inner surface of femur 15 to drill a bone tunnel 25 up into femur 15. More particularly, once tibial tunnel 20 is created, a conventional femoral guide, often referred to as an "over-the-top" guide (Fig. 4), is used to accurately locate the femoral tunnel 25. More specifically, the "over-the-top" guide is placed through the tibial tunnel, across the joint, through the femoral notch, and then into position so that the distal finger of the guide is positioned against the backside of the femur. (Fig. 5).

Proper placement of the femoral tunnel is imperative in order for the ACL graft to be properly positioned on the femur. However, as a result of using the aforementioned trans-tibial technique and the aforementioned conventional "over-the-top" femoral guide, the position of the femoral tunnel is effectively dictated by the position of the first-drilled tibial tunnel. This often results in a femoral tunnel position, and thus, an ACL reconstruction (i.e., graft orientation, etc.) that is less than optimal.

In an attempt to better position the femoral tunnel, surgeons have recently begun utilizing the so-called "medial portal technique" to drill and create the femoral tunnel. By drilling the femoral tunnel through the medial portal or an accessory portal, the femoral and tibial tunnels may be drilled independently of one another and, therefore, in a more appropriate anatomical position.

As shown in Fig. 6, when drilling the femoral tunnel through the medial portal, surgeons typically still use the same "over-the-top" femoral guide used during the aforementioned trans-tibial approach. However, because the "over-the-top" femoral guide is designed for use in a trans-tibial approach, the "over-the-top" femoral guide is not ideal for use in a medial portal approach. These "over-the-top" femoral guides generally have narrow-shaped distal tip geometries to aid in their ability to pass through the tibial tunnel. In addition, such femoral guides have an offset spatula design to hook the posterior femoral notch, thereby aiding in positioning of the guide. Aside from this spatula design, these femoral guides have no other specific referencing geometries for properly positioning the femoral tunnel.

Traditionally, surgeons utilize what is known as a "clock face" orientation in order to decide where to place the femoral tunnel within the notch of knee. This clock face orientation technique designates positions along the notch from 9 o'clock to 3 o'clock, depending on which knee is being reconstructed. This technique, while seemingly simplistic, is limited by a number of factors, one being that the positioning of the imaginary clock face along the notch is completely subjective and hence widely affected by the specific implementation of the surgeon.

Therefore, it would be beneficial to have a femoral guide for use in medial approach ACL reconstruction surgery that is configured for more accurate femoral tunnel positioning. In addition, it would be beneficial if the femoral guide is designed in such a way that it might also be utilized during a trans-tibial approach.

### SUMMARY

A surgical apparatus for reconstruction of a ligament according to claim 1 is provided.

The distal tip further may further include at least one of opposed fingers and a distal projection. The opposed fingers or distal projection may be configured to reference a leading edge of the posterior cruciate ligament. The opposed fingers or distal projections may further be configured to reference a posterior femoral cortex. The elongated body may be configured to extend across a knee joint, the length of a tibial tunnel, or out of a medial port. The distal end may include a substantially circular cross-section, a substantial semi-spherical cross-section, or an unroofed cross-section.

Additionally, provided is method of positioning a femoral tunnel during an ACL reconstruction, the method not forming part of the invention. The method includes the steps of providing a femoral guide including an elongated body having a distal end, the distal end including a diameter substantially similar in size to the diameter of the desired resultant femoral tunnel, wherein the elongated body and the distal end are cannulated to receive a guide wire therethrough, inserting the femoral guide into a knee joint, positioning the distal end of the guide against the femur, and inserting the guide wire through the femoral guide and into the femur.

The femoral guide may include one of opposed fingers and distal projection configured for referencing a posterior cruciate ligament. The method may further include the step of referencing a leading edge of a posterior cruiciate ligament and/or the posterior femoral cortex. The method may also include the step of flexing the knee to 120 degrees. The femoral guide may be inserted into the knee joint using a medial portal approach or a trans-tibial approach.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a perspective view of a knee joint showing an ACL;
FIG. 2 is an alternate perspective view of the knee joint of FIG. 1;
FIG. 3 is a perspective view of a knee joint including tibial and femoral tunnels (shown in phantom) and a ligament graft;
FIG. 4 is a side view of a conventional "over-the-top" femoral guide;
FIG. 5 is side view of a knee joint including the "over-the-top" femoral guide of
FIG. 4 accessing the femur using the trans-tibial approach;
FIG. 6 is a side view of a knee joint including the "over-the-top" femoral guide of FIG. 4 access the femur using the medial portal approach;

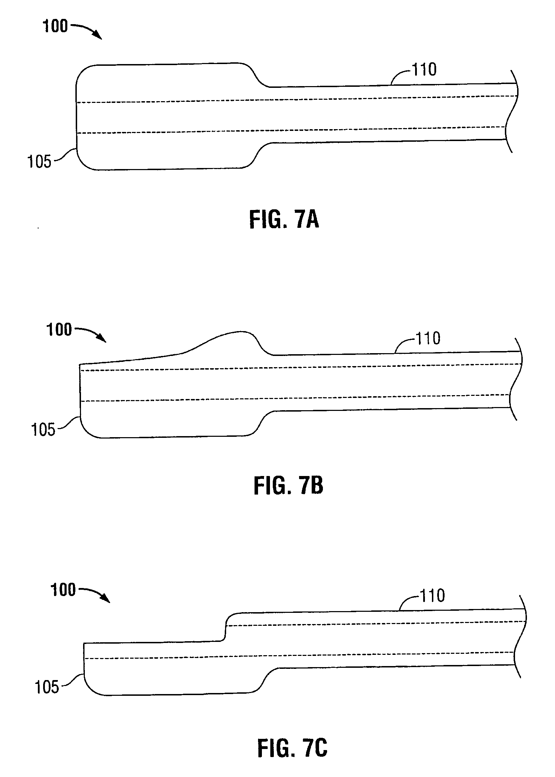

FIGS. 7A-7C are side views of the distal end of various femoral guides;

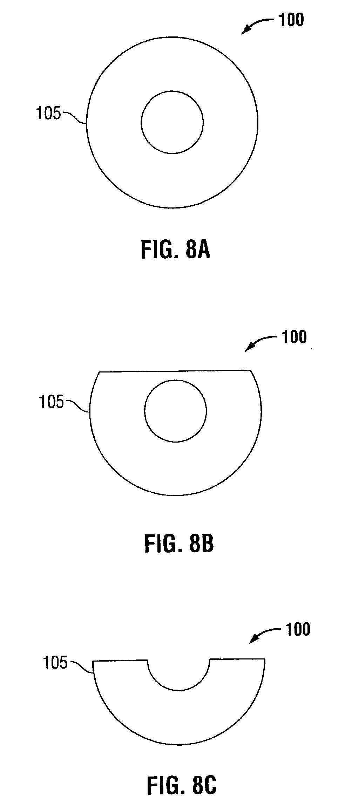

FIGS. 8A-8C are end views of the distal end of the guides of FIGS. 7A-7C, respectively;

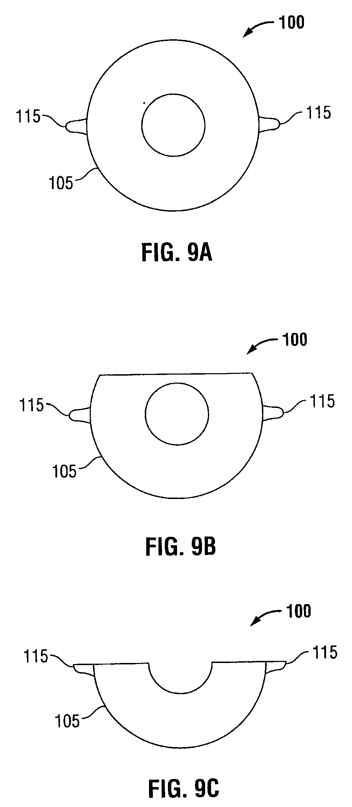

FIGS. 9A-9C are end views of the distal end of femoral guides;

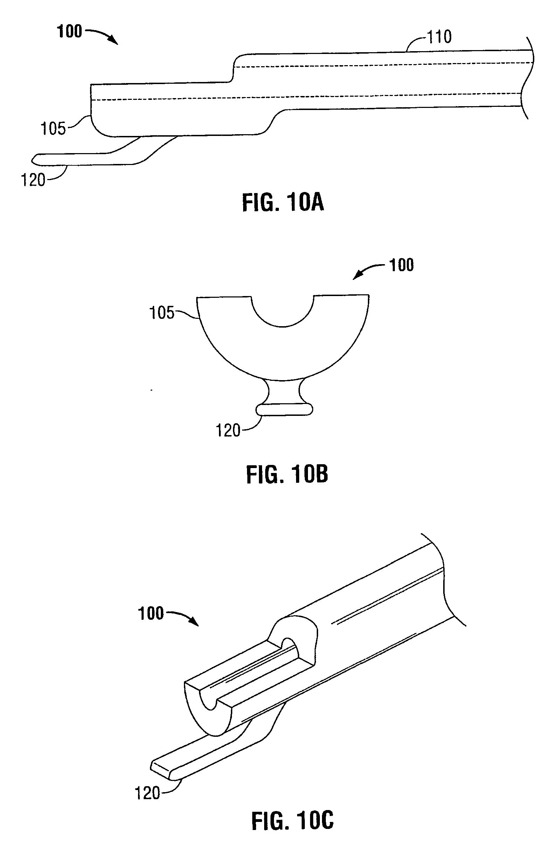

FIG. 10A is a side view of a distal end of a femoral guide;
FIG. 10B is an end view of the distal end of the femoral guide of FIG. 10A;
FIG. 10C is a side view of the femoral guide of FIGS. 10A and 10B;

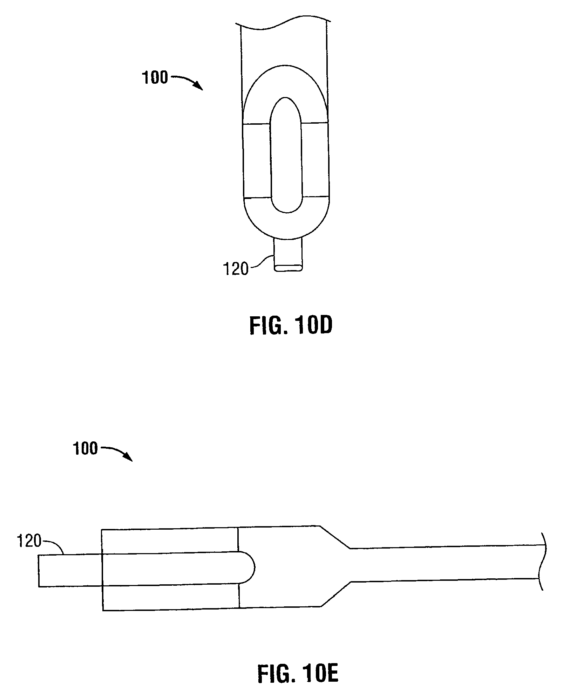

FIG. 10D is an end view of the femoral guide of FIGS. 10A-10C;
FIG. 10E is top view of the distal end of the femoral guide of FIGS. 10A-10D;

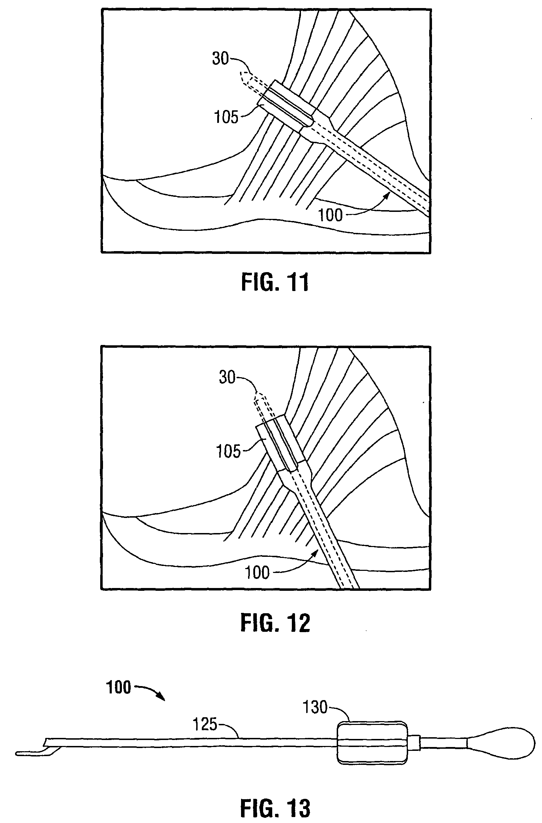

FIG. 11 is a partial cut-away view of a femoral guide being used in a medial portal approach;
FIG. 12 is a partial cut-away view of a femoral guide being used in a trans-tibial approach;
FIG. 13 is a side view of the proximal end of a femoral guide.

### DETAILED DESCRIPTION OF THE EMBODIMENTS

The femoral guide of the present disclosure is designed to be used in determining the position of a femoral tunnel guide wire which facilitates position of a femoral tunnel during an ACL reconstruction. As with conventional femoral guides, the femoral guide of the present disclosure may reference an "over-the-top" position with an offset spatula; however, it can also be designed and utilized without such an offset spatula. This includes having no spatula, or instead having one or multiple spike projections or other similar projections to hold the spatula in position on the bone.

Figs. 7A-10E show various femoral guides 100. Femoral guide 100 generally includes a distal tip 105 and a shaft 110 extending proximally therefrom. Distal tip 105 of femoral guide 100 is dimensioned and configured to have the same geometry and circumference as the desired resulting femoral tunnel. In this manner, distal tip 105 acts as a visual aid to assist the surgeon in determining proper tunnel placement by providing a direct visual confirmation of where the resulting femoral tunnel will be located. Alternatively but not forming part of the invention, distal tip 105 may be formed with a semi-hemispherical cross-section (FIGS. 7B, 8B and 9B), or with an "unroofed" cross-section (FIGS. 7C, 8C, 9C and 10A-E) to aid in visualization.

Shaft 110 of femoral guide 100 is configured to be of such a length so as to at least extend (i) across the knee joint, (ii) across the length of the tibial tunnel and/or (iii) out of the medial portal. Shaft 110 and distal tip 105 are cannulated so as to accept (and thereby aim) a guidewire of an appropriate circumference, length and width.

In addition, the geometry of distal end 105 of femoral guide 100 may include (i) diametrically-opposed fingers 115 (FIGS. 9A-9C), and/or (ii) a distal projection 120 (FIGS. 10A-10E). Fingers 115 and/or projection 120 serve to reference the leading edge of the posterior cruciate ligament (PCL) and the posterior femoral cortex. Using the PCL as an anatomical reference enables a surgeon to set the femoral guide wire, and therefore the resulting femoral tunnel, in a position that better avoids any impingement of the PCL after the graft ligament has been placed in position. Such ACL/PCL impingement occurs when the femoral tunnel has been improperly positioned. In this manner, femoral guide 100 is configured to avoid any such ACL/PCL impingement, by using the PCL as an anatomical reference during formation of the femoral tunnel.

As shown in Fig. 11, femoral guide 100 is used in a medial portal approach with the knee in hyper-flexion, at approximately 120 degrees. However, it should be appreciated that femoral guide 100 may also be used with any ACL reconstruction approach, and with any angle of knee flexion. See, for example, Fig. 12, where femoral guide 100 is used during a traditional trans-tibial approach. Because of the size and/or configuration of distal end 105 of femoral guide 100, for use in the trans-tibial approach, femoral guide 100 may be halved, with one half for use with the right knee and the other half for use with the left knee.

Once the location of femoral tunnel 25 is identified by the surgeon with distal end 105 of femoral guide 100, guide wire 30 (FIG. 11) is extended through the cannulated shaft of elongated body 110 and into femur 15. Once guide wires 30 has been inserted into femur 15 to a desired depth, femoral guide 100 is then removed from about guide wire 30 and from the medial portal into the knee. A cannulated drill bit (not shown) is then received about guide wire 30 and through the medial portal to drill femoral tunnel 25.

Looking next at Fig. 13, the proximal (or "butt") end 125 of femoral guide 100 is preferably provided with a docking port 130 to mate with a handle 135 to aid the surgeon in aiming the guide more easily and accurately. Handle 135 may be configured in any desired geometry so as to be ergonomically comfortable and/or to facilitate in the placement or holding of distal tip 105 in a particular position.

Femoral guide 100 provides surgeons with several significant improvements over prior art femoral guides. First, the distal portion of femoral guide 100 is configured (both in shape and diameter), to mirror that of the resulting tunnel and, therefore, the resulting graft. This gives the surgeon a visual "preview" or reference of the femoral tunnel prior to actually drilling the femoral tunnel. In addition, the distal shape of the femoral guide references the leading edge of the PCL's insertion onto the femur (i.e., the location where the PCL attaches to the femur) and places the resulting femoral tunnel in a position which avoids graft ACL/PCL impingement.

It should be understood that many additional changes in the details, materials, steps and arrangements of parts, which have been herein described and illustrated in order to explain the nature of the present disclosure, may be made by those skilled in the art while still remaining within the principles and scope of the invention as defined in the claims.

## Claims

1. A surgical apparatus for reconstruction of a ligament comprising:
a guide (100) for positioning a guide wire on a femur (15), the guide comprising a guide wire (30);
a cannulated drill bit for drilling a femoral tunnel about the guide wire; and
an elongated body (110) having proximal and distal ends, the elongated body being cannulated to receive the guide wire; wherein the distal end of the guide has a distal tip (105), the distal tip having a greater diameter than the elongated body and being cannulated to receive the guide wire;
**characterized in that**
the distal tip is dimensioned and configured to have the same geometry and circumference as the cannulated drill bit whereby the distal tip has the same geometry and circumference as the resultant femoral tunnel drilled by the drill bit.

2. The surgical apparatus of claim 1, wherein the distal tip of the guide further includes at least one of opposed fingers (115) and a distal projection (120).

3. The surgical apparatus of claim 1, wherein the elongated body of the guide is configured to extend across a knee joint.

4. The surgical apparatus of claim 1, wherein the elongated body of the guide is configured to extend the length of a tibial tunnel (20).

5. The surgical apparatus of claim 1, wherein the elongated body of the guide is configured to extend out of a medial port.

6. The surgical apparatus of claim 1, wherein the distal end of the guide includes a substantially circular cross-section.

## Patentansprüche

1. Chirurgische Vorrichtung zur Rekonstruktion eines Ligaments, umfassend:
- eine Führung (100) zur Positionierung eines Führungsdrahtes an einem Oberschenkelknochen (15), wobei die Führung einen Führungsdraht (30) aufweist;
- einen kanülierten Bohreinsatz zum Bohren eines femoralen Tunnels um den Führungsdraht; und
- einen langgestreckten Körper (110), der proximale und distale Enden aufweist; wobei der langgestreckte Körper kanüliert ist, um den Führungsdraht aufzunehmen; wobei das distale Ende der Führung eine distale Spitze (105) aufweist, wobei die distale Spitze einen größeren Durchmesser als der langgestreckte Körper aufweist und kanüliert ist, um den Führungsdraht aufzunehmen;
- **dadurch gekennzeichnet, dass**
- die distale Spitze dimensioniert und konfiguriert ist, dass sie die gleiche Gestaltung und den gleichen Umfang wie der kanülierte Bohreinsatz aufweist, wodurch die Spitze die gleiche Gestaltung und den gleichen Umfang wie der sich ergebende femorale Tunnel aufweist, der durch den Bohreinsatz gebohrt wurde.

2. Chirurgische Vorrichtung nach Anspruch 1, wobei die distale Spitze der Führung ferner mindestens einen von einander gegenüberliegenden Fingern (115) und einen distalen Vorsprung (120) aufweist.

3. Chirurgische Vorrichtung nach Anspruch 1, wobei der langgestreckte Körper der Führung konfiguriert ist, sich über ein Kniegelenk zu erstrecken.

4. Chirurgische Vorrichtung nach Anspruch 1, wobei der langgestreckte Körper der Führung konfiguriert ist, sich über die Länge eines tibialen Tunnels (20) zu erstrecken.

5. Chirurgische Vorrichtung nach Anspruch 1, wobei der langgestreckte Körper der Führung konfiguriert ist, sich aus einer medialen Öffnung heraus zu erstrecken.

6. Chirurgische Vorrichtung nach Anspruch 1, wobei das distale Ende der Führung einen im Wesentlichen kreisförmigen Querschnitt aufweist.

## Revendications

1. Appareil chirurgical pour la reconstruction d'un ligament comprenant :
un guide (100) pour positionner un fil de guidage sur un fémur (15), le guide comprenant un fil de guidage (30) ;
une mèche canulée pour forer un tunnel fémoral autour du fil de guidage ; et
un corps allongé (110) ayant des extrémités proximale et distale, le corps allongé étant canulé pour recevoir le fil de guidage ; dans lequel l'extrémité distale du guide présente une pointe distale (105), la pointe distale ayant un diamètre supérieur à celui du corps allongé et étant canulée pour recevoir le fil de guidage ;
**caractérisé en ce que** :
la pointe distale est dimensionnée et configurée pour avoir la même géométrie et la même circonférence que la mèche canulée, si bien que la pointe distale a la même géométrie et la même circonférence que le tunnel fémoral obtenu foré par la mèche.

2. Appareil chirurgical selon la revendication 1, dans lequel la pointe distale du guide comprend en outre au moins l'un ou l'autre de doigts opposés (115) et d'une saillie distale (120).

3. Appareil chirurgical selon la revendication 1, dans lequel le corps allongé du guide est configuré pour s'étendre en travers d'une articulation du genou.

4. Appareil chirurgical selon la revendication 1, dans lequel le corps allongé du guide est configuré pour étendre la longueur d'un tunnel tibial (20).

5. Appareil chirurgical selon la revendication 1, dans lequel le corps allongé du guide est configuré pour s'étendre hors d'un orifice médian.

6. Appareil chirurgical selon la revendication 1, dans lequel l'extrémité distale du guide comprend une section transversale sensiblement circulaire.
